(19) Europäisches Patentamt
European Patent Office
Office européen des brevets

(11) Numéro de publication: **0 016 665**
**B1**

# (12) FASCICULE DE BREVET EUROPEEN

(45) Date de publication du fascicule de brevet: **05.05.82**

(51) Int. Cl.³: **C 07 D 311/86** // C07C149/40, C07C149/425, C07C143/82

(21) Numéro de dépôt: **80400146.9**

(22) Date de dépôt: **30.01.80**

(54) **Procédé de préparation de dérivés de la xanthone.**

(30) Priorité: **09.02.79 GB 7904649**

(43) Date de publication de la demande:
**01.10.80 Bulletin 80/20**

(45) Mention de la délivrance du brevet:
**05.05.82 Bulletin 82/18**

(84) Etats contractants désignés:
**AT BE CH DE FR IT LU NL SE**

(56) Documents cités:
**FR-A-2 277 576**
**FR-A-2 423 487**
**CHEMICAL ABSTRACTS, vol. 90, n° 17, 23 avril 1979, pages 20–21, n° 132 583k**
**Columbus, Ohio, USA**
**A.C. BARNES et al.: «Pharmacologically active sulfoximides: 5-hexyl-7-(S-methylsulfonimidoyl)xanthone-2-carboxylic acid, a potent antiallergic agent»**

(73) Titulaire: **ROUSSEL-UCLAF, 35, boulevard des Invalides, F-75007 Paris (FR)**

(72) Inventeur: **Barnes, Alan Charles, 12 St. Peter's Road, Cirencester Gloucestershire (GB)**
Inventeur: **Kennewell, Peter David, 10 St. Helens's View, Swindon Wiltshire (GB)**

(74) Mandataire: **Vieillefosse, Jean-Claude et al, ROUSSEL-UCLAF Boîte postale no. 9 102, route de Noisy, F-93230 Romainville (FR)**

**EP 0 016 665 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

# Procédé de préparation de dérivés de la xanthone

La présente invention concerne un nouveau procédé de préparation de dérivés de la xanthone.

L'invention a pour objet un nouveau procédé de préparation des composés de formule générale I:

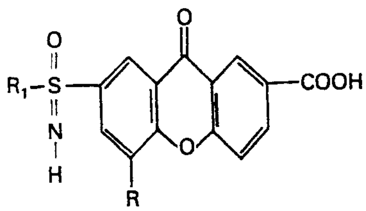

(I)

dans laquelle R représente un atome d'hydrogène ou un radical alcoyle renfermant de 1 à 9 atomes de carbone et $R_1$ représente un radical alcoyle renfermant de 1 à 5 atomes de carbone.

Les composés de formule générale I, ainsi que certains procédés pour les préparer sont décrits dans le brevet français n° 2 277 576.

Ainsi qu'il est indiqué dans ce brevet, les composés de formule générale I possèdent une intéressante activité antiallergique et sont, en particulier, utiles dans le traitement de l'asthme et des bronchites asthmatiformes d'origine allergique.

Un nouveau procédé de préparation des composés de formule générale I a ensuite été décrit dans la demande française publiée, n° 2 423 487.

Dans la formule générale I, R représente, de préférence, un radical n-hexyle et $R_1$ un radical méthyle.

Le nouveau procédé de la présente invention permet de préparer les composés de formule générale I avec de bons rendements en un nombre restreint de stades et utilise des réactifs d'emploi plus aisé que les procédés précédents.

L'invention a ainsi pour objet un procédé de préparation des composés de formule générale I, telle que définie ci-dessus, caractérisé en ce que l'on fait réagir un phénol de formule:

(II)

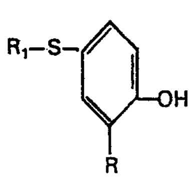

dans laquelle R et $R_1$ ont la signification déjà indiquée, avec un halogénure d'acyle de formule Hal–CO–X dans laquelle Hal représente un atome de chlore ou de brome et X représente un radical alcoyle renfermant de 1 à 3 atomes de carbone ou un radical phényle ou avec un anhydride d'acide de formule $(X{-}CO)_2O$ dans laquelle X a la signification déjà indiquée, pour obtenir un produit de formule:

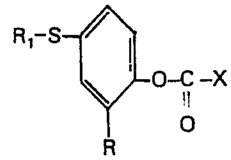

(III)

dans laquelle X, R et $R_1$ ont la signification déjà indiquée, fait réagir ce dernier avec un réactif de formule

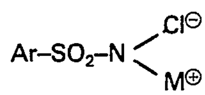

dans laquelle Ar représente un radical aryle éventuellement substitué et M représente un atome de métal alcalin, pour obtenir un produit de formule:

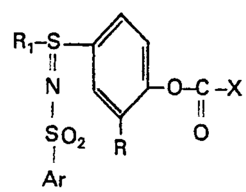

(IV)

dans laquelle X, R, $R_1$ et Ar ont la signification déjà indiquée, oxyde le produit de la formule IV ainsi obtenu, pour obtenir un produit de formule:

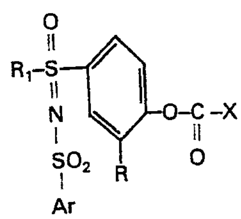

(V)

dans laquelle X, R, $R_1$ et Ar ont la signification déjà indiquée, hydrolyse ce dernier pour obtenir un produit de formule:

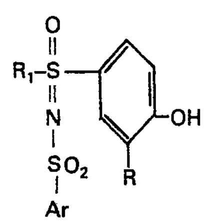

(VI)

dans laquelle R, $R_1$ et Ar ont la signification déjà indiquée, fait réagir le produit de formule VI obtenu, en présence d'une base faible et de cuivre métallique ou d'oxyde de cuivre, avec un isophtalate d'alcoyle de formule:

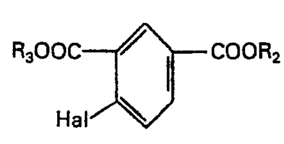

(VII)

dans laquelle $R_2$ et $R_3$, identiques ou différents, représentent un groupement estérifiant et Hal représente un atome de chlore, de brome ou d'iode, pour obtenir un produit de formule:

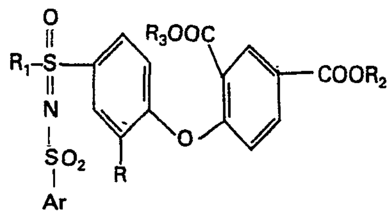
(VIII)

dans laquelle R, $R_1$, $R_2$, $R_3$ et Ar ont la signification déjà indiquée, puis cyclise le produit de formule VIII ainsi obtenu, en opérant en présence d'un acide fort, pour obtenir, le cas échéant après hydrolyse, le produit de formule I recherché.

Dans la formule générale IV, Ar représente, de préférence, un radical aryle monocyclique renfermant de 6 à 10 atomes de carbone, par exemple, un radical phényle, ce radical aryle pouvant être substitué par un ou plusieurs radicaux alcoyles renfermant de 1 à 4 atomes de carbone tels des radicaux méthyles. Le radical para-tolyle est le radical préféré.

– Le réactif de formule 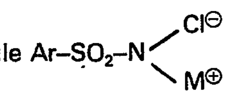

est de préférence la chloramine T (p-tolyl-

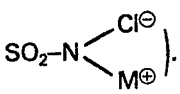).

Dans la formule générale VII, les groupements estérifiants $R_2$ et $R_3$ peuvent, par exemple, représenter un radical alcoyle renfermant de 1 à 3 atomes de carbone, un radical aryle, un radical aralcoyle ou un radical dialcoylaminoalcoyle.

– Le radical aryle peut être notamment un radical nitrophényle; le radical aralcoyle peut être notamment un radical nitrobenzyle; le radical dialcoylaminoalcoyle peut être notamment un radical diméthylaminoéthyle.

Les groupements estérifiants que représentent $R_2$ et $R_3$ sont choisis, de préférence, de telle façon que le composé de formule VIII soit obtenu sous forme cristallisée ce qui facilite sa purification, tel le groupement méthyle.

Dans des conditions préférentielles de mise en œuvre du procédé de l'invention:

a) La réaction de l'halogénure d'acyle ou de l'anhydride d'acide avec le phénol de formule II est effectuée au sein d'un solvant organique anhydre, tel que la pyridine.

b) La réaction de la chloramine avec le produit de formule III est effectuée au sein d'un solvant organique anhydre et de préférence d'un alcool, tel que l'alcool isopropylique.

c) L'oxydation du produit de formule IV est effectuée au moyen du métaperiodate de sodium et de l'oxyde de ruthénium au sein d'un solvant organique anhydre, tel que le dichlorométhane.

On peut aussi opérer en présence d'un peroxyde. Les periodates étant normalement utilisés en solution aqueuse, on peut donc utiliser dans le cas présent, un système solvant à deux phases.

d) L'hydrolyse du produit de formule V est effectuée au moyen d'une solution aqueuse d'une base faible telle que le carbonate de sodium, en opérant à une température d'ébullition du mélange réactionnel.

e) La réaction du produit de formule VI avec le produit de formule VII est effectuée en présence d'une base faible telle que le carbonate de sodium ou de potassium et de cuivre métallique sous forme de poudre, en opérant au reflux du mélange.

f) La cyclisation est effectuée à une température pouvant aller de 100 à 150°, en présence d'un acide fort tel que l'acide polyphosphorique ou l'acide sulfurique concentré. L'hydrolyse du composé obtenu se fait alors, en général, simultanément ou encore, par exemple, par addition d'eau. Cependant, si le composé cyclisé est obtenu sous forme estérifiée, l'hydrolyse peut être effectuée de manière indépendante selon les méthodes connues.

Les exemples suivants illustrent l'invention sans toutefois la limiter.

Exemple 1: Acide 5-hexyl-7-(S-méthyl sulfonimidoyl) xanthone-2-carboxylique.

Stade A: Benzoate de 2-hexyl-4-(méthylthio)phényl

On agite pendant deux heures à température ambiante une solution de 75 g de 2-hexyl-4-(méthylthio)phénol dans 225 $cm^3$ de pyridine et 100 g de chlorure de benzoyl, on ajoute ensuite 450 $cm^3$ d'eau et agite le mélange pendant une heure. On filtre le produit cristallisé formé, le lave à l'eau, le sèche et obtient 110 g de produit attendu. F = 57–58 °C.

Stade B: Benzoate de 2-hexyl-4-(S-méthyl-N-tosyl sulfimidoyl)phényl

On dissout 43 g de chloramine T dans un litre d'isopropanol à 50 °C, puis ajoute en 15 minutes, 50 g du produit obtenu au stade A. On agite pendant 15 minutes, filtre, refroidit à 0 °C et laisse cristalliser. Le produit formé est essoré, est séché, on obtient ainsi 63 g de produit attendu. F = 111–112 °C.

Stade C: Benzoate de 2-hexyl-4-(s-méthyl-N-tosyl sulfonimidoyl phényl

On dissout 50 g du produit obtenu au stade B dans 250 $cm^3$ de chlorure de méthylène puis ajoute 100 mg d'oxyde de ruthénium et 50 $cm^3$ d'une solution aqueuse de périodate de sodium à 10%. On agite vigoureusement, une émulsion vert pâle se forme, celle-ci prend une teinte foncée après quelques minutes lorsque le périodate a été consommé, le processus est répété à quatre reprises avec à chaque fois 50 $cm^3$ de la solution aqueuse de périodate de sodium, la quantité totale de périodate de sodium utilisée a été finalement de 25 g. On sépare la phase organique, extrait la phase aqueuse au chlorure de méthylène, lave les phases organiques réunies à l'eau, les sèche, les concentre à sec. On obtient ainsi une huile brute qui est utilisée telle quelle pour le stade suivant.

L'huile obtenue peut être cristallisée F. 90–91 °C.

Stade D: 2-hexyl-4-(S-méthyl-N-tosyl sulfonimidoyl)phénol

On dissout l'huile obtenue au stade C dans 250 $cm^3$ de méthanol puis ajoute à la solution obtenue 500 $cm^3$ d'une solution aqueuse à 10% de carbonate de sodium et agite à 60 °C pendant trois heures.

On refroidit ensuite à température ambiante puis ajoute 100 $cm^3$ d'acide chlorhydrique à 20% et extrait au chloroforme; Après évaporation du solvant sous pression réduite et après avoir trituré le résidu dans l'éther de pétrole, on obtient 40 g de produit attendu. F = 109–110 °C.

Stade E: 4-/2-hexyl-4-(S-méthyl-N-tosyl sulfonimidoyl)phénoxy/isophtalate de diméthyl.

On mélange 20 g de produit obtenu au stade D avec 15 g de 4-bromoisophtalate de diméthyl, 10 g de carbonate de potassium, 1 g de cuivre en poudre, 100 $cm^3$ de nitrobenzène et 50 $cm^3$ de toluène, puis on agite sous gaz inerte, en chauffant lentement jusqu'à 160 °C en distillant environ 25 $cm^3$ de toluène.

On maintient la température à 160 °C pendant deux heures puis évapore le reste du solvant, refroidit le résidu, le dissout dans 400 $cm^3$ de chlorure de méthylène, filtre et évapore à sec. On dissout le résidu dans 250 $cm^3$ d'éther, refroidit à 0 °C et cristallise le produit.

On filtre, lave à l'éther, sèche et obtient 15,7 g de produit attendu. F = 124–126 °C. Par concentration des liqueurs-mères, on obtient un deuxième jet de 6,8 g de produit brut.

Stade F: acide 5-hexyl-7-(méthyl sulfonimidoyl)-xanthone-2-carboxylique

On dissout 5 g du produit obtenu au stade E dans 25 $cm^3$ d'acide sulfurique concentré qui chauffe à 120 ° pendant une heure. On refroidit et verse sous agitations dans 250 $cm^3$ d'eau. On filtre les cristaux formés, les lave à l'eau, les recristallise dans le méthanol et obtient 2,5 g de produit attendu. F = 193–194 °C.

Exemple 2: Acide 5-hexyl-7-(S-méthyl sulfonimidoyl)xanthone-2-carboxylique

Stade A: Acétate de 2-hexyl-4-(méthylthio)phényl

On agite pendant une nuit à température ambiante une solution de 6,6 g de 2-hexyl-4-(méthylthio phénol dans 36 $cm^3$ de pyridine et 18 $cm^3$ d'anhydride acétique. On verse ensuite dans l'eau, extrait à l'éther, lave la phase organique avec une solution aqueuse diluée d'acide chlorhydrique puis avec une solution aqueuse de bicarbonate de sodium et enfin à l'eau, sèche et évapore à sec. On obtient 7,45 g de produit attendu.

Stade B: Acétate de 2-hexyl-4-(S-méthyl-N-tosyl sulfimidoyl)phényl.

On agite pendant 5 heures une solution de 5 g du produit obtenu au stade A et 6,5 g de trihydrate de chloramine T dans 250 $cm^3$ de dioxanne et 125 $cm^3$ d'eau. On évapore ensuite à sec et obtient 11 g d'un résidu huileux. On chromatographie ce résidu sur silice en éluant au chloroforme. On obtient 5 g de produit attendu que l'on cristallise dans l'éther de pétrole. F = 62–65 °C.

Stade C: Acétate de 2-hexyl-4- (S-méthyl-N-tosyl sulfonimidoyl)phényl

On dissout 1 g du produit obtenu au stade B dans 40 $cm^3$ de chlorure de méthylène puis ajoute 1 g de métapériodate de sodium dans l'eau et 5 mg d'oxyde de ruthénium.

On agite le mélange pendant 30 minutes puis ajoute à nouveau 5 mg d'oxyde de ruthénium et 0,3 g de métapériodate de sodium. On agite pendant 15 minutes, sépare la phase organique, filtre et évapore à sec. Le résidu est chromatographié sur silice en éluant à l'acétate d'éthyle.

On obtient 0,88 g du produit attendu sous forme d'une huile qui se solidifie en refroidissant.

Stade D: 2-hexyl-4-(S-méthyl-N-tosyl sulfonimidoyl)phénol

On met en suspension 0,8 g du produit obtenu au stade C dans 30 $cm^3$ d'une solution saturée de carbonate de sodium et 15 $cm^3$ d'éthanol.

On agite à 50 °C pendant 15 minutes, refroidit la solution obtenue dans l'eau, acidifie par addition d'acide chlorhydrique concentré et extrait à l'éther.

On décante la phase organique, évapore le solvant et obtient 0,65 g de produit attendu qui cristallise dans le mélange éther/éther de pétrole. F = 107–109 °C.

Stade E: 4-/2-hexyl-4-(S-methyl-N-tosyl sulfonimidoyl)phénoxy/isophtalate de diméthyle.

On dissout 0,4 g du produit obtenu au stade D et 0,3 g de bromoisophtalate de diméthyle dans 10 $cm^3$ de nitrobenzène, sèche le mélange par distillation azéotropique avec du benzène, ajoute à la solution 50 mg de cuivre en poudre, 0,4 g de carbonate de potassium anhydre et agite pendant deux heures à 160 °C sous gaz inerte. On refroidit le mélange, dilue au chloroforme, filtre et évapore le solvant sous pression réduite. On triture le résidu huileux dans l'éther de pétrole et obtient 0,53 g de produit attendu cristallisé. On chromatographie sur silice en éluant au chloroforme, on obtient des cristaux blancs de produit attendu. F = 115–117 °C.

Stade F: 5-hexyl-7-(S-méthyl sulfonimidoyl)xanthone-2-carboxylate de méthyle.

On dissout 0,2 g du produit obtenu au stade E dans 4 $cm^3$ d'acide polyphosphorique, agite à 80 °C pendant 10 minutes puis augmente la température jusqu'à 120 °C.

On maintient l'agitation à 120 °C pendant une heure et demie puis refroidit, verse dans l'eau et

extrait à l'acétate d'éthyle. On évapore le solvant et obtient 0,11 g du produit qui se révèle être un mélange de 5-hexyl-7-(S-méthyl sulfonimidoyl)-xanthone-2-carboxylate de méthyle et de l'acide correspondant.

On obtient l'ester pur par chromatographie sur silice en éluant à l'acétate d'éthyle.

Stade G: Acide 5-hexyl-7-(S-méthyl sulfonimidoyl)xanthone-2-carboxylique.

On dissout 0,1 g de l'ester obtenu au stade F dans 5 $cm^3$ d'éthanol et 1 $cm^3$ d'eau puis ajoute 2,7 $cm^3$ d'une solution 0,2N d'hydroxyde de sodium dans l'eau. On porte au reflux pendant une heure, refroidit, acidifie la solution par addition d'acide chlorhydrique 2N puis essore les cristaux formés, les lave à l'eau puis les sèche.
On obtient 0,09 g de produit attendu. F = 193–194 °C.

**Revendications:**

1. Procédé de préparation des composés de formule générale I:

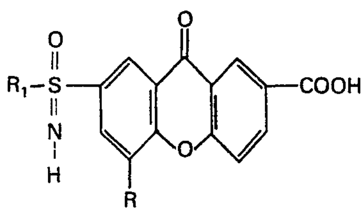 (I)

dans laquelle R représente un atome d'hydrogène ou un radical alcoyle renfermant de 1 à 9 atomes de carbone et $R_1$ représente un radical alcoyle renfermant de 1 à 5 atomes de carbone, caractérisé en ce que l'on fait réagir un phénol de formule:

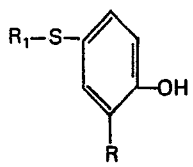 (II)

dans laquelle R et $R_1$ ont la signification déjà indiquée, avec un halogénure d'acyle de formule Hal–CO–X dans laquelle Hal représente un atome de chlore ou de brome et X représente un radical alcoyle renfermant de 1 à 3 atomes de carbone ou un radical phényle ou avec un anhydride d'acide de formule $(X{-}CO)_2O$ dans laquelle X a la signification déjà indiquée, pour obtenir un produit de formule:

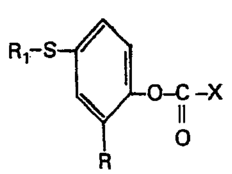 (III)

dans laquelle X, R et $R_1$ ont la signification déjà indiquée, fait réagir ce dernier avec un réactif de formule:

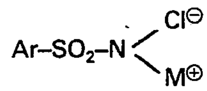

dans laquelle Ar représente un radical aryle éventuellement substitué et M représente un atome de métal alcalin, pour obtenir un produit de formule:

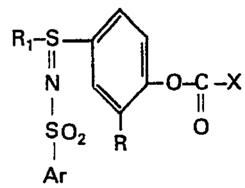 (IV)

dans laquelle X, R, $R_1$ et Ar ont la signification déjà indiquée, oxyde le produit de la formule IV ainsi obtenu, pour obtenir un produit de formule:

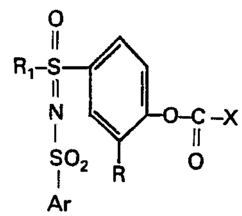 (V)

dans laquelle X, R, $R_1$ et Ar ont la signification déjà indiquée, hydrolyse ce dernier pour obtenir un produit de formule:

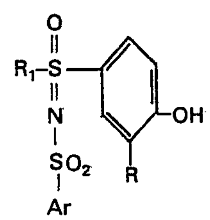 (VI)

dans laquelle R, $R_1$ et Ar ont la signification déjà indiquée, fait réagir le produit de formule VI obtenu, en présence d'une base faible et de cuivre métallique ou d'oxyde de cuivre, avec un isophtalate d'alcoyle de formule:

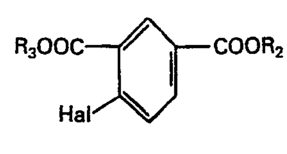

dans laquelle $R_2$ et $R_3$, identiques ou différents, représentent un groupement estérifiant, et Hal représente un atome de chlore, de brome ou d'iode, pour obtenir un produit de formule:

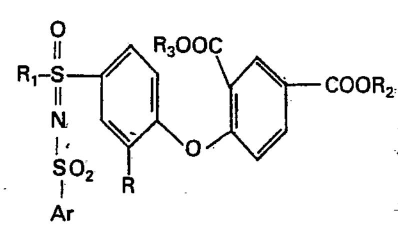 (VIII)

dans laquelle R, $R_1$, $R_2$, $R_3$ et Ar ont la signification déjà indiquée, puis cyclise le produit de formule VIII ainsi obtenu, en opérant en présence d'un acide fort pour obtenir, le cas échéant après hydrolyse, le produit de formule I recherché.

2. Procédé selon la revendication 1, caractérisé en ce que l'on utilise au départ un composé de formule II, dans laquelle R représente un radical n-hexyle et $R_1$ un radical méthyle.

3. Procédé selon la revendication 1 ou 2, caractérisé en ce que dans le réactif que l'on fait agir avec le composé de formule III, Ar représente un radical p-tolyle.

4. Procédé selon l'une quelconque des revendications 1, 2 ou 3, caractérisé en ce que dans le composé de formule VII, $R_2$ et $R_3$, identiques ou différents, représentent un radical alcoyle renfermant de 1 à 3 atomes de carbone, un radical aryle, un radical aralcoyle ou un radical dialcoylaminoalcoyle.

5. Procédé selon l'une quelconque des revendications 1, 2, 3 ou 4, caractérisé en ce que:

a) la réaction de l'halogénure d'acyle ou de l'anhydride d'acide avec le phénol de formule II est effectuée au sein d'un solvant organique anhydre tel que la pyridine;

b) la réaction de la chloramine avec le produit de formule III est effectuée au sein d'un solvant organique anhydre tel que l'alcool isopropylique;

c) l'oxydation du produit de formule IV est effectuée au moyen du métaperiodate de sodium et de l'oxyde de ruthénium au sein d'un solvant organique anhydre, tel que le dichlorométhane;

d) l'hydrolyse du produit de formule V est effectuée au moyen d'une solution aqueuse d'une base faible telle que le carbonate de sodium, en opérant à la température d'ébullition du mélange réactionnel;

e) la réaction du produit de formule VI avec le produit de formule VII est effectuée en présence d'une base faible telle que le carbonate de sodium ou de potassium et de cuivre métallique sous forme de poudre, en opérant au reflux du mélange;

f) la cyclisation est effectuée à une température pouvant aller de 100 à 150 °C, en présence d'un acide fort, tel que l'acide polyphosphorique ou l'acide sulfurique concentré.

**Patentansprüche**

1. Verfahren zur Herstellung von Verbindungen der allgemeinen Formel I:

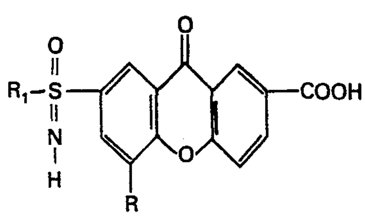

(I)

worin R ein Wasserstoffatom oder einen Alkylrest mit 1 bis 9 Kohlenstoffatomen bedeutet und $R_1$ einen Alkylrest mit 1 bis 5 Kohlenstoffatomen darstellt, dadurch gekennzeichnet, dass man ein Phenol der Formel:

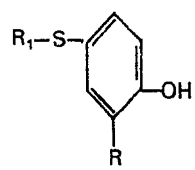

(II)

worin R und $R_1$ die angegebene Bedeutung besitzen, mit einem Acylhalogenid der Formel Hal–CO–X, worin Hal ein Chlor- oder Bromatom bedeutet und X einen Alkylrest mit 1 bis 3 Kohlenstoffatomen oder einen Phenylrest darstellt, oder mit einem Säureanhydrid der Formel $(X{-}CO)_2O$, worin X die vorstehend angegebene Bedeutung besitzt, umsetzt, um ein Produkt der Formel:

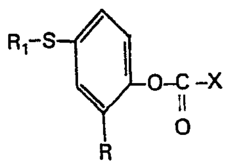

(III)

zu erhalten, worin X, R und $R_1$ die vorstehend angegebene Bedeutung besitzen, dieses letztere mit einem Reagens der Formel:

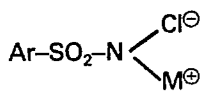

worin Ar einen gegebenenfalls substituierten Arylrest darstellt und M ein Alkalimetallatom bedeutet, umsetzt, um ein Produkt der Formel:

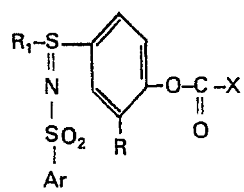

(IV)

zu erhalten, worin X, R, $R_1$ und Ar die angegebene Bedeutung besitzen, das so erhaltene Produkt der Formel IV oxidiert, um ein Produkt der Formel:

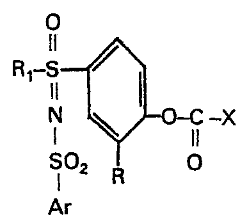

(V)

zu erhalten, worin X, R, $R_1$ und Ar die angegebene Bedeutung besitzen, dieses letztere hydrolysiert, um ein Produkt der Formel:

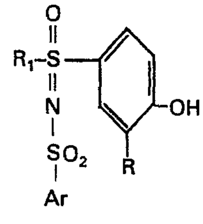

(VI)

zu erhalten, worin R, $R_1$ und Ar die angegebene Bedeutung besitzen, das erhaltene Produkt der Formel VI in Gegenwart einer schwachen Base und von metallischem Kupfer oder Kupferoxid mit einem Alkylisophthalat der Formel:

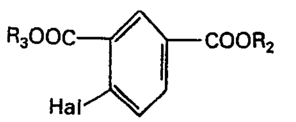 (VII)

worin $R_2$ und $R_3$, die gleich oder verschieden sein können, eine veresternde Gruppe bedeuten und Hal ein Chlor-, Brom- oder Jodatom bedeutet, umsetzt, um ein Produkt der Formel:

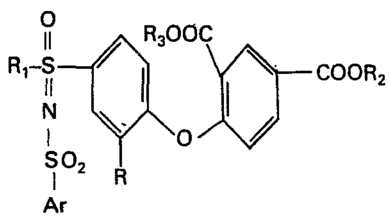 (VIII)

zu erhalten, worin R, $R_1$, $R_2$, $R_3$ und Ar die angegebene Bedeutung besitzen, danach das so erhaltene Produkt der Formel VIII cyclisiert, wobei man in Gegenwart einer starken Säure arbeitet, um gegebenenfalls nach Hydrolyse das gewünschte Produkt der Formel I zu erhalten.

2. Verfahren gemäss Anspruch 1, dadurch gekennzeichnet, dass man von einer Verbindung der Formel II ausgeht, worin R einen n-Hexylrest bedeutet und $R_1$ einen Methylrest darstellt.

3. Verfahren gemäss Anspruch 1 oder 2, dadurch gekennzeichnet, dass in dem Reagens, das man mit der Verbindung der Formel III umsetzt, Ar einen p-Tolylrest bedeutet.

4. Verfahren gemäss einem der Ansprüche 1, 2 oder 3, dadurch gekennzeichnet, dass in der Verbindung der Formel VII $R_2$ und $R_3$, die gleich oder verschieden sein können, einen Alkylrest mit 1 bis 3 Kohlenstoffatomen, einen Arylrest, einen Aralkylrest oder einen Dialkylaminoalkylrest bedeuten.

5. Verfahren gemäss einem der Ansprüche 1, 2, 3 oder 4, dadurch gekennzeichnet, dass

a) die Umsetzung des Acylhalogenids oder des Säureanhydrids mit dem Phenol der Formel II in dem Medium eines Wasserfreien organischen Lösungsmittels, wie Pyridin, durchgeführt wird;

b) die Umsetzung des Chloramins mit dem Produkt der Formel III in dem Medium eines wasserfreien organischen Lösungsmittels, wie Isopropylalkohol, durchgeführt wird;

c) die Oxidation des Produkts der Formel IV mit Hilfe von Natrium-metaperjodat und Rutheniumoxid in dem Medium eines wasserfreien organischen Lösungsmittels, wie Methylenchlorid, durchgeführt wird;

d) die Hydrolyse des Produktes der Formel V mit Hilfe einer wässrigen Lösung einer schwachen Base, wie Natriumcarbonat, durchgeführt wird, wobei man bei der Siedetemperatur des Reaktionsgemisches arbeitet;

e) die Umsetzung des Produktes der Formel VI mit dem Produkt der Formel VII in Gegenwart einer schwachen Base, wie Natrium- oder Kaliumcarbonat, und von metallischem Kupfer in Pulverform durchgeführt wird, wobei man unter Rückfluss des Gemisches arbeitet;

f) die Cyclisierung bei einer Temperatur von 100 bis 150 °C in Gegenwart einer starken Säure, wie Polyphosphorsäure oder konzentrierte Schwefelsäure, durchgeführt wird.

**Claims:**

1. Process for preparing the compounds of general formula I:

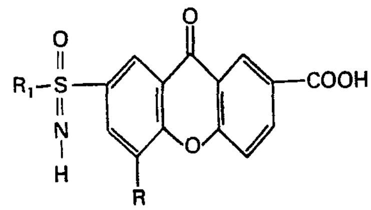 (I)

in which R represents a hydrogen atom or an alkyl radical containing form 1 to 9 carbon atoms and $R_1$ represents an alkyl radical containing from 1 to 5 carbon atoms, characterised in that a phenol of formula:

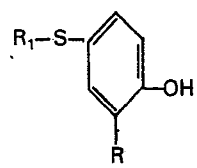 (II)

in which R and $R_1$ have the meaning already indicated, is reacted with an acyl halide of formula Hal–CO–X in which Hal represents a chlorine or bromine atom and X represents an alkyl radical containing from 1 to 3 carbon atoms or a phenyl radical or with an acid anhydride of formule $(X{-}CO)_2O$ in which X has the meaning already indicated, to obtain a product of formula:

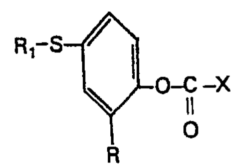 (III)

in which X, R and $R_1$ have the meaning already indicated, the latter is reacted with a reagent of formula:

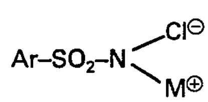

in which Ar represents an aryl radical, possibly substituted, and M represents an alkali metal atom, to obtain a product of formula:

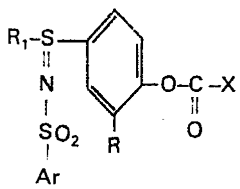

(IV)

in which X, R, $R_1$ and Ar have the meaning already indicated, the product of the formula IV thus obtained is oxidized, to obtain a product of formula:

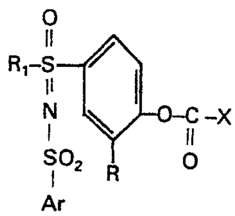

(V)

in which X, R, $R_1$ and Ar have the meaning already indicated, the latter is hydrolysed to obtain a product of formula:

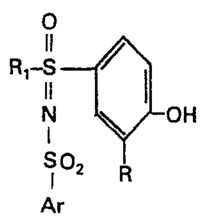

(VI)

in which r, $R_1$ and Ar have the meaning already indicated, the product of formula VI obtained is reacted, in the presence of a weak base and of metallic copper or of copper oxide, with an alkyl isophthalate of formula:

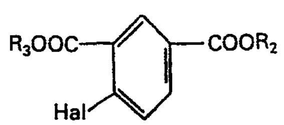

(VII)

in which $R_2$ and $R_3$, being the same or different, represent an esterifying group and Hal represents a chlorine, bromine or iodine atom, to obtain a product of formula:

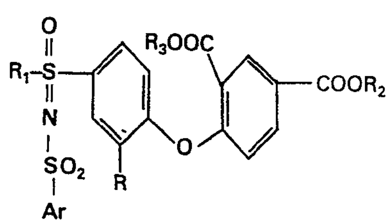

(VIII)

in which R, $R_1$ $R_2$, $R_3$ and Ar has the meaning already indicated, then the product of formula VIII thus obtained is cyclised, work being carried out in the presence of a strong acid to obtain, if necessary after hydrolysis, the product of formula I sought.

2. Process according to claim 1, characterised in that to start with a compound is used of formula II in which R represents a n-hexyl radical and $R_1$ a methyl radical.

3. Process according to claim 1 or 2, characterised in that in the reagent which is reacted with the compound of formula III, Ar represents a p-tolyl radical.

4. Process according to any one of claims 1, 2 or 3, characterised in that, in the compound of formula VII, $R_2$ and $R_3$, being the same or different, represent an alkyl radical containing from 1 to 3 carbon atoms, an aryl radical, an aralkyl radical or a dialkylaminoalkyl radical.

5. Process according to any one of claims 1, 2, 3 or 4, characterised in that:

a) the reaction of the acyl halide or of the acid anhydride with the phenol of formula II is carried out in an anhydrous organic solvent such as pyridine;

b) the reaction of the chloramine with the product of formula III is carried out in an anhydrous organic solvent such an isopropyl alcohol;

c) the oxidation of the product of formula IV is carried out by means of sodium metaperiodate and of ruthenium oxide in an anhydrous organic solvent such as dichloromethane;

d) the hydrolysis of the product of formula V is carried out by means of an aqueous solution of a weak base such as sodium carbonate, work being carried out at the boiling temperature of the reaction mixture;

e) the reaction of the product of formula VI with the product of formula VII is carried out in the presence of a weak base such as sodium carbonate or potassium carbonate and of metallic copper in the form of powder, work being carried out at reflux of the mixture; and

f) the cyclisation is carried out at a temperature capable of ranging from 100 to 150 °C, in the presence of a strong acid, such as polyphosphoric acid or concentrated sulphuric acid.